# EUROPÄISCHE PATENTSCHRIFT

(11) **EP 0 682 922 B1**
(45) Veröffentlichungstag und Bekanntmachung des Hinweises auf die Patenterteilung: **09.04.1997**
(21) Anmeldenummer: 94106490.9
(22) Anmeldetag: 26.04.1994
(51) Int. Cl.: A61F 2/04

(54) **Selbstexpandierender Stent für Hohlorgane**
Self-expanding stent for hollow organs
Dilatateur auto-expansible d'organes creux

(43) Veröffentlichungstag der Anmeldung: 22.11.1995
(73) Patentinhaber: WILLY RÜSCH AG, D-71394 Kernen (DE)
(72) Erfinder: Schmitt, Klaus, D-73630 Remshalden-Grunbach (DE); Singvogel, Armin, D-71686 Remseck am Neckar 2 (DE); Entenmann, Helmuth, D-73614 Schorndorf-Schornbach (DE); Amstrup, Mogens, D-73614 Schorndorf (DE); Klepetko, Walter, A-1090 Wien (AT)
(74) Vertreter: KOHLER SCHMID + PARTNER

(56) Entgegenhaltungen:
- EP-A- 0 221 570
- EP-A- 0 556 850
- WO-A-91/12779
- DE-B- 1 766 921
- US-A- 5 141 502

## Beschreibung

Gegenstand der Erfindung ist ein Stent zur Schienung und/oder zum Offenhalten eines Hohlorgans, mit aus Filamenten bestehendem tubulärem und selbstexpandierbarem Netz, das im kollabierten Zustand in Achsrichtung gestreckt und im ausgedehnten Zustand in Achsrichtung verkürzt ist, wobei die Filamente im Querschnitt rechteckförmig ausgebildet sind.

Ein derartiger Stent ist durch die DE-B-17 669 21 bekanntgeworden.

Stents bekannter Art werden z. B. zur Behandlung von Gefäßleiden eingesetzt. Stents ermöglichen, ein Gefäßlumen permanent offen zu halten, in dem sie transluminal implantiert werden. Mit Hilfe der interventionellen Radiologie können somit chirurgische Eingriffe ergänzt oder im günstigsten Fall überflüssig werden. Die transluminale Applikation von Stents zur Schienung oder permanenten Dehnung von Stenosen ist besonders bei Patienten mit erhöhtem chirurgischem Risiko vorteilhaft.

Zur Schienung und zum Offenhalten einer Gefäßverengung sind neben Metallmemoryprothesen, die aus einem Nitioldraht bestehen, Doppelhelixspiralen bekannt, die eine Lumeneinengung dadurch weiten, indem sie über ein bekanntes Einführungsinstrument an die entsprechende Gefäßstelle geführt und in situ expandiert werden. Die Doppelhelixspirale wird durch die eigene Expansionskraft gegen die Gefäßwand in situ gehalten.

Weiterhin ist ein "Palmaz-Stent" bekannt, der aus einem dünnwandigen Rohr aus rostfreiem Stahl mit Längsschlitzen besteht. Der rohrförmige Stent ist zum Einführen auf einen Dilatationsballon montiert und wird in situ durch Insufflation des Ballons ausgedehnt und abgelöst. Durch die Expansion des rohrförmigen Stents wird eine netzartige Struktur ausgebildet.

Der aus der EP 0 177 330 B1 bekannte Stent besteht aus einem Stahldraht, der Zick-Zack-förmig ausgeformt ist. Der Stent läßt sich zusammengepresst durch einen im Gefäß plazierten Katheter vorschieben, wobei sich der Stent nach dem Ausstoßen durch die Eigenspannung von selbst ausdehnt. Die Eigenspannung des bekannten Stents kann durch die Wahl der Stärke des Runddrahts, dem Grad der Vorbiegung und der Anzahl der Zick-Zack-Biegungen variiert werden. Nachteilig wirken sich in diesem System die möglichen Materialermüdungsbrüche des Runddrahtes im Bereich der besonders biegungsbeanspruchten "Z-Anteile" aus.

Die Auslegeschrift DE-B-17 669 21 beschreibt einen als Stent verwendbaren chirurgischen Dilatator, der ein gitterartiges Rohr besitzt, dessen Gitterelement so ausgebildet sind und/oder so verlaufen, daß sich die Wandung des Rohres beim Einwirken einer axialen Zugkraft diametral zusammenzieht und axial ausdehnt und beim Wegfall der Zugkraft im wesentlichen wieder ihre normale Form annimmt. Das Gitterrohr bzw. dessen Wandung ist in eine durchgehende Schicht von elastischem Material eingebettet und das Gitterrohr besteht aus aneinander angelehnten Flachbändern die von Fäden so unter Spannung gehalten werden, daß sie das Bestreben haben, den Dilatator im Durchmesser zu erweitern.

Der Erfindung liegt die Aufgabe zugrunde, einen selbstexpandierenden Stent der eingangs genannten Art dahingehend weiterzubilden, daß bei verbessertem Expansionsverhalten eine möglichst schonende Anlage der Filamente an der Innenwandung des Hohlorgans gewährleistet und eine Entfernung des plazierten Stents leichter ermöglicht wird.

Diese Aufgabe wird erfindungsgemäß durch die Merkmale des Anspruchs 1 gelöst.

Der erfindungsgemäße Stent hat damit den Vorteil, daß er kollabiert einen kleineren Durchmesser aufweisen kann wie die bekannten selbstexpandierenden Stents. Eine Vereinfachung und eine Reproduzierbarkeit in der Herstellung des erfindungsgemäßen Stents wird auch dadurch erreicht, daß er einfach nachvollziehbar geflochten oder gewoben werden kann. Die Lage des einzelnen und gewundenen Filaments kann über die gesamte Länge des Stents optisch einfachst nachvollzogen werden.

Ferner lassen sich die Kreuzungspunkte einfach und definiert miteinander fixieren, weil eine flächenhafte Auflage der Filamente an den Kreuzungspunkten gegeben ist. Eine undefinierte Verspleisung der Filamentenden und damit eine Verwucherung der Endbereiche bei bekannten Stents mit dem angrenzenden Gefäßgewebe kann beim erfindungsgemäßen Stent nicht auftreten, weil die einzelnen Enden der Filamente miteinander fixiert sind. Weiterhin ist vorteilhaft, daß die flächenhaften Filamente von Röntgenstrahlen sehr gut erfaßbar sind und die gesamte Lage des plazierten Stent deutlich nachweisbar ist. Wird bei einer gewünschten Entfernung des erfindungsgemäßen Stents ein fester Kreuzungspunkt an jedem Ende aufgefunden, so kann der Stent gedehnt und in bekannter Weise wie eingeführt auch aus dem Gefäß oder aus dem Hohlorgang entfernt werden.

Zum Aufbau des erfindungsgemäßen Stents haben sich Flachbandfilamente bewährt. Wird für die Herstellung des erfindungsgemäßen Stents ein Stahlflachband benützt, das wesentlich breiter als hoch ist, so ist der Stent im gestreckten Zustand sehr dünn und kann über sehr dünne Einführungsinstrumente sicher perkutan oder durch das Lumen des Hohlorgans eingeführt und plaziert werden. Die Einführung und Plazierung sowie die Selbstexpansion im Kohlorgan bzw. im Gefäß sind damit sehr einfach. Expandiert der erfindungsgemäße Stent, so liegt er flächenhaft an der Innenwandung des Hohlorgans an. Die Gefahr der Perforation der Hohlorganwand wird durch die flächenhafte Anlage der einzelnen Filamente reduziert.

Weiterhin weist der erfindungsgemäße Stent eine große Variabilität im Durchmesser, in der Länge und in der Expansionsstärke bei großer longitudinaler Flexibilität auf. Dies wird dadurch erreicht, daß bei einem Flachband die unterschiedlich gerichteten Kraftkomponenten auch unterschiedlich groß sind. Bei einem Runddraht sind die aus ihm resultierenden Kraftkomponenten immer gleich groß, weil bei einem im Querschnitt kreisförmigen Filament die aus dem Material wirkenden Kräfte nicht wesentlich unterschiedlich sind. Der erfindungsgemäße Stent kann sich damit den unterschiedlichsten Verläufen der Hohlorgane anpassen. Durch die flächenhafte Anlage an der Innenwandung des zu dehnenden Hohlorgans kann auch die Trombogenität und eine eventuelle überschießende intimale Hyperplasie im Bereich der Stentenden reduziert werden.

In bevorzugter Ausgestaltung der Erfindung sind die Filamente von einem gummielastischen Kunststoffmaterial ummantelt. Dies trägt zu einer verbesserten Biokompatibilität bei.

Im ausgedehnten Zustand des erfindungsgemäßen Stents sind zwischen den Filamenten Freiräume ausgebildet.

Bei dem erfindungsgemäßen Stent sind die Filamente entgegen der Grundbiegung zu einem tubulären Netz flecht- oder webbar. Dies hat den Vorteil, daß die Flexibilität und der Expansionsdruck, d.h. die Eigenspannung des erfindungsgemäßen Stents, mittels einer Variation der Netzdichte und der Flachbandauswahl (Metall, Breite, Höhe) definiert variiert werden kann.

In einer weiteren Ausgestaltung der Erfindung sind die einzelnen Filamente neben einer gummielastischen Beschichtung aus Kunststoff noch mit einer zusätzlichen hydrophilen Beschichtung ausgestattet. Dies ermöglicht verbesserte Durchflußwerte an der aufgedehnten Stelle im Hohlorgan.

Der erfindungsgemäße Stent kann mit einem bekannten Einführungsinstrument plaziert werden und ist in diesem Einführungsinstrument lösbar angeordnet. Dies hat den Vorteil, daß schon bestehende Einführungsinstrumente auch für die Plazierung des erfindungsgemäßen Stents verwendet werden können.

Weitere Vorteile ergeben sich aus der Beschreibung und der beigefügten Zeichnung. Ebenso können die vorstehend genannten und die noch weiter aufgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter. Die Erfindung ist in der Zeichnung dargestellt und wird anhand von Ausführungsbeispielen erläutert. Es zeigt:
- Fig. 1: einen erfindungsgemäßen Stent in gestrecktem Zustand, wobei die erfindunsgemäße Kunststoffummantelung der Kreuzungspunkte der Filamente zur Verbesserung der Übersichtlichkeit nicht dargestellt ist.
- Fig. 1a: ein einzelnes Filament als Metallflachband in ebener gerader und gebogener Erstreckung;
- Fig. 1b: einen Querschnitt gemäß Ib-Ib der Fig. 1a;
- Fig. 2: einen Stentabschnitt im ausgedehnten Zustand mit Kreuzungspunkten, die gelenkig miteinander verbunden sind;
- Fig. 3: eine weitere Ausführungsform eines Stentabschnitts im ausgedehnten Zustand mit verbundenen Kreuzungspunkten, die von sich kreuzenden Filamenten gebildet sind;
- Fig. 3a: eine Ausschnittsvergrößerung eines Stents im selbstexpandierten Zustand an den Kreuzungspunkten;
- Fig. 3b: eine Ausschnittsvergrößerung eines Stents im aktivlängsgestreckten Zustand an den Kreuzungspunkten;
- Fig. 4: einen Stent im Bereich eines freien Endes.

Die einzelnen Figuren der Zeichnung zeigen den erfindungsgemäßen Gegenstand teilweise stark schematisiert und sind nicht maßstäblich zu verstehen. Die Gegenstände der einzelnen Figuren sind stark vergrößert dargestellt, damit ihr Aufbau besser gezeigt werden kann.

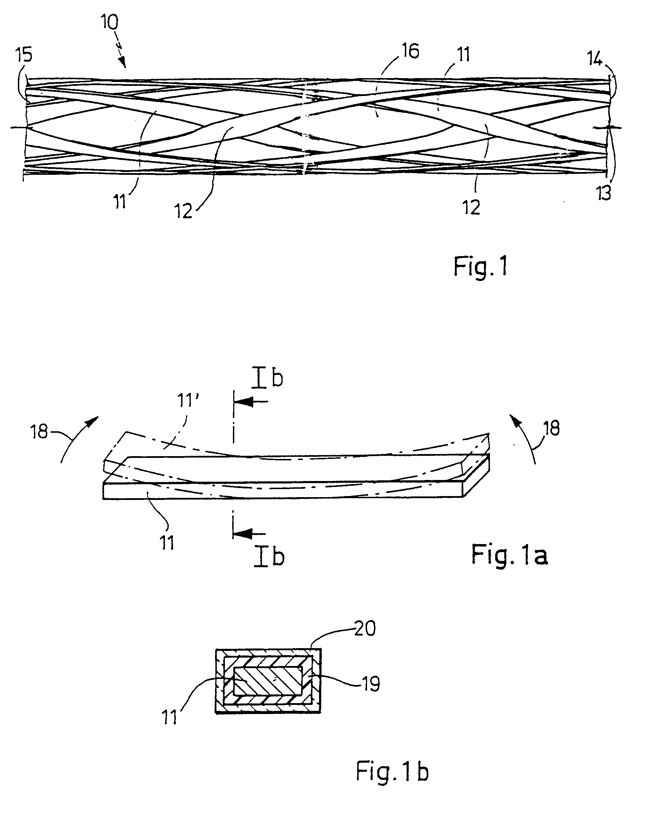

Fig. 1 zeigt mit 10 einen teilweise kollabierten Stent, der aus Filamenten 11 gebildet ist (zwei Filamente sind in der Figur mit dem Bezugszeichen 11 versehen), die aus Flachdrahtbändern bestehen. Die Flachdrahtbänder weisen beispielsweise eine Breite von 0,075 mm auf und sind 0,005 mm dick. Die Metallflachbänder sind beispielsweise aus rostfreiem Stahl gefertigt. An Kreuzungspunkten 12 liegen die Filamente 11 übereinander und weisen zusammen die doppelte Höhe eines einzelnen Filaments 11 auf. An den Kreuzungspunkten 12 liegen die Filamente 11 flächenhaft übereinander. Mehrere Filamente 11 sind zu einem tubulären Netz geflochten, das den Stent 10 bildet. Bezüglich einer Achse 13 sind die Filamente 11 längsgestreckt. An den freien Enden 14, 15 sind die Kreuzungspunkte 12 derart ausgeführt, daß die einzelnen sich kreuzenden Filamente 11 fest miteinander fixiert sind. Zwischen den einzelnen Filamenten 11 sind rautenförmige Freiräume 16 ausgebildet, die je nach Dehnungszustand des Stents 10 bzw. ausgedehntem Zustand des Stents 10 mehr oder weniger groß sein können. An den Kreuzungspunkten 12 liegen die Filamente 11 über ihre Metallflächen aufeinander und der gesamte Kreuzungspunkt 12 ist kunststoffummantelt (der Übersichtlichkeit wegen nicht dargestellt).

Fig. 1a zeigt ein Filament 11, wie es zum Stent 10 in der Fig. 1 mehrfach gewunden verwendet wurde. Das Filament 11 ist ein rechteckförmig ausgebildetes Stahlflachband, das eine Grundbiegung aufweist, die in der Fig. gestrichelt dargestellt ist. Das Filament 11 biegt sich aufgrund der Herstellung in Pfeilrichtungen 18 zu einem Filament 11'.

Fig. 1b zeigt einen Schnitt Ib-Ib der Fig. 1a. Das Filament 11, ein Stahlflachband, ist von einem elastischen Kunststoffmaterial 19 vollkommen ummantelt. Weiterhin ist das elastische Kunststoffmaterial 19 vollkommen von einer hydrophilen Schicht 20 umgeben, die je nach Bedarf bei den einzelnen Ausbildungsformen des erfindungsgemäßen Stents vorhanden ist. Es kann auch vorteilhaft sein, daß an den Auflagen in den Kreuzungspunkten der einzelnen Filamente 11 das elastische Kunststoffmaterial 19 ausgespart ist, damit die Gesamtdicke des Netzes an dem Kreuzungspunkt 12 reduziert wird.

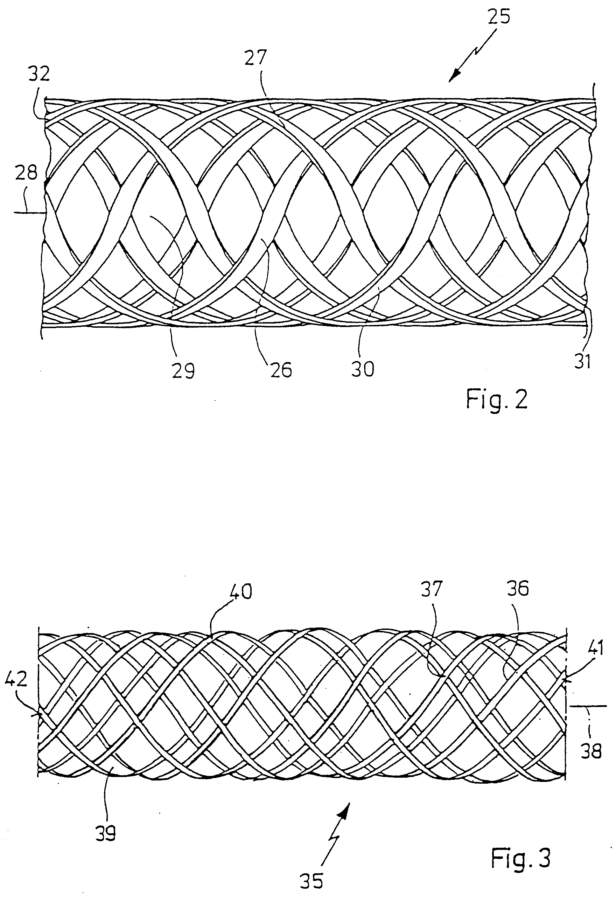

Fig. 2 zeigt einen Stentabschnitt 25 im ausgedehnten Endzustand. Filamente 26 haben sich gemäß ihrer Vorspannung ausgedehnt und um Kreuzungspunkte 27 derart gedreht, daß Freiräume 29 entstehen, die um ein Vielfaches größer sind als beispielsweise die Freiräume 16, wie sie in Fig. 1 gezeigt sind. Gegenüber einer Achse 28 ist der Stentabschnitt 25 gekürzt und mit 30 ist die gelenkartige Verbindung gezeigt, wie die einzelnen Kreuzungspunkte 27 der Filamente 26 zusammengehalten sind. Die Filamente 26 sind an den Kreuzungspunkten vollkommen ummantelt und innerhalb der ummantelten Schicht können sich die Filamente 26 zueinander gelenkig bewegen. Je nach dem, welches Material für die Ummantelung ausgewählt wird, kann eine Rückstellkraft aus dem kollabierten Zustand des erfindungsgemäßen Stents zum ausgedehnten Zustand verstärkt werden. In der Endlage, wie sie beispielsweise in Fig. 2 gezeigt ist, weist der Stent im ausgedehnten Zustand einen Außendurchmesser von ca. 3 mm bis ca. 30 mm auf. Die Größe der Endlage richtet sich nach der Stärke der ausgewählten Metallflachbänder, der Flecht- bzw. Webweise und der Art der Kunststoffummantelung der einzelnen Filamente 26. Gegenüber einer Achse 28 ist der Stentabschnitt 25 logitudinal gesehen im aufgedehnten Zustand verkürzt. Die freien Enden des Stents 25 sind in der Figur mit den Bezugszeichen 31, 32 gekennzeichnet.

Fig. 3 zeigt eine weitere Ausführungsform eines Stentabschnitts 35 im ausgedehnten Zustand. Dabei sind die im Querschnitt rechteckförmigen Filamente 36 zu einem tubulären Netz geflochten. Die Filamente 36 kreuzen sich an Kreuzpunkten 37. Gegenüber einem in der Fig. 3 nicht gezeigten kollabierten Zustand des Stentabschnitts 35 ist der in der Fig. gezeigte Stentabschnitt 35 bezogen auf die Achse 38 longitudinal gesehen verkürzt. Die aneinander grenzenden Abschnitte der Filamente 36 begrenzen Freiräume 39, die rautenförmig ausgebildet sind und in die sich angrenzendes Gewebe der Hohlorgane eindrücken kann. Mit 40 ist ein Gelenk gezeigt, bei dem die einzelnen Filamente 36 lose aufeinanderliegen. Aufgrund der Vorspannung im netzartigen Geflecht drücken sich die einzelnen Filamente 36 aneinander. Die Vorspannung ist im Stent vorgegeben, so daß das tubuläre Netz durch eine radiale Vergrößerung in einen definierten Endzustand übergeht. Im ausgedehnten Zustand wird immer ein- und dieselbe Lumenweite aufrecht erhalten. Dabei paßt sich der erfindungsgemäße Stent an Bewegungen, die radial bzw. longitudinal gerichtet sind, bestmöglich an. Freie Ende des Stents 35 sind mit 41, 42 gekennzeichnet. Die Filamente 36 sind im Bereich der freien Enden 41, 42 in den Kreuzungspunkten 37 fest miteinander verbunden.

Fig. 3 a zeigt Filamente 45, die mit einem Kunststoff 46 gummielastischer Art ummantelt sind. Dabei sind die Filamente 45 gestrichelt dargestellt. An Kreuzungspunkten 47 liegen die Filamente 45 über ihre metallischen Flächen aneinander an und die sich kreuzenden Filamente 45 sind gesamthaft kunststoffummantelt, sodaß sich ein fixiertes Gelenk im Kreuzungspunkt 47 bildet. In der Fig. 3 a ist der Kreuzungspunkt 47 in einer selbstexpandierten Stellung gezeigt. Dabei ist der den Kreuzungspunkt 47 ummantelnde Kunststoff 46 nahezu spannungsfrei.

Fig. 3 b zeigt die Filamente 45 mit einer Kunststoffummantelung 46 im aktiv längsgestreckten Zustand. Im Kreuzungspunkt 47 ist der Kunststoff in Pfeilrichtung 48 gedehnt und in Pfeilrichtungen 49 komprimiert.

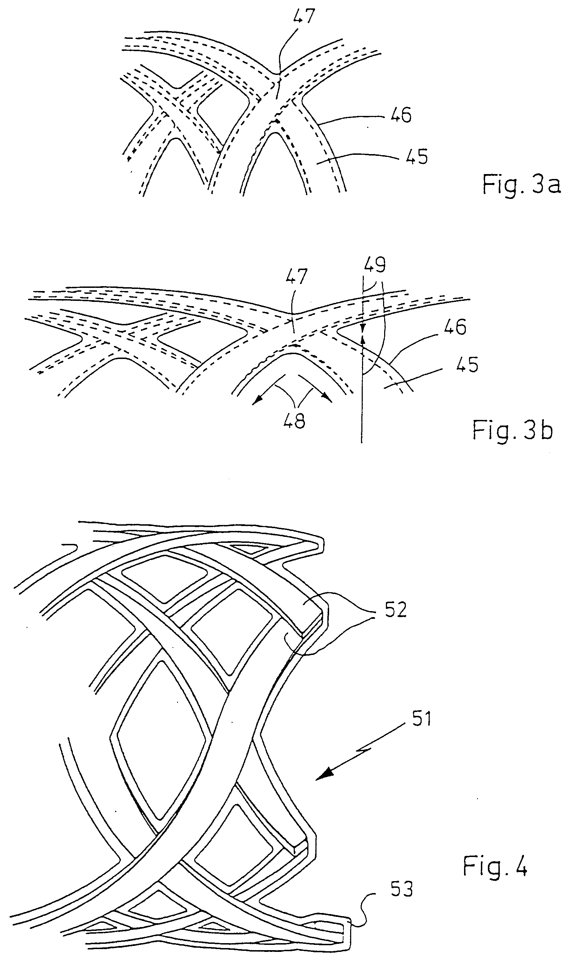

Fig. 4 zeigt ein freies Ende 51 eines erfindungsgemäßen Stents. Dabei sind die freien Enden von Filamenten 52 übereinanderliegend fixiert, indem sie entweder kunststoffummantelt sind, wie dies mit dem Bezugszeichen 53 gezeigt ist. Die Auflager können auch noch zusätzlich über beispielsweise eine Verlötung miteinander fixiert sein. Die flächenhaften Auflager lassen sich sicher fixieren. Auch eine Verklebung sich kreuzender Filamentenden ist denkbar.

Der in der Fig. 1 bis 4 gezeigte Stent ist von Röntgenstrahlen sehr gut erfaßbar und kann damit deutlich nachgewiesen werden.

Ein Stent zur Schienung und/oder zum Offenhalten eines Hohlorgans besteht aus Filamenten 11, die zu einem tubulären und selbstexpandierbaren Netz zusammengefügt sind. Im kollabierten Zustand ist der Stent 10 in Achsrichtung gestreckt und im ausgedehnten Zustand ist der Stent 10 in Achsrichtung verkürzt. Die Filamente 11 sind im Querschnitt rechteckförmig ausgebildet und die Kreuzungspunkte 12 sind an den freien Enden 14 und 15 fest miteinander verbunden.

## Patentansprüche

1. Stent zur Schienung und/oder zum Offenhalten eines Hohlorgans, mit aus Filamenten (11; 11'; 26; 36; 45) bestehendem tubulärem und selbstexpandierbarem Netz, das im kollabierten Zustand in Achsrichtung (13) gestreckt und im ausgedehnten Zustand in Achsrichtung (28; 38) verkürzt ist, wobei die Filamente (11; 11'; 26; 36; 45) im Querschnitt rechteckförmig ausgebildet und an Kreuzungspunkten (12; 27; 37; 47) des Stents (10; 25; 35) gesamthaft mit einem Kunststoff (46) gummielastischer Art ummantelt sind, um ein fixiertes Gelenk im Kreuzungspunkt (12; 27; 37; 47) zu bilden, wobei zwischen den Filamenten (11; 11'; 26; 36; 45) Freiräume (16; 29; 39) ausgebildet sind.

2. Stent nach Anspruch 1, dadurch gekennzeichnet, daß das Netz aus Drahtfilamenten, insbesondere aus Metall, aufgebaut ist.

3. Stent nach einem der Ansprüche 1 oder 2, dadurch gekennzeichnet, daß die Filamente von einem gummielastischen Kunststoffmaterial (19) ummantelt sind.

4. Stent nach einem der Ansprüche 1 bis 3, dadurch gekennzeichnet, daß die Filamente (11; 11'; 26; 36) entgegen der Grundbiegung zu einem tubulären Netz flecht- oder webbar sind.

5. Stent nach einem der Ansprüche 1 bis 4, dadurch gekennzeichnet, daß die Filamente (11; 11'; 26; 36) mit einer hydrophilen Beschichtung (20) versehen sind.

6. Stent nach einem der Ansprüche 1 bis 5, dadurch gekennzeichnet, daß der Stent (10; 25; 35) an einem Einführungsinstrument lösbar angeordnet ist.

## Claims

1. Stent for splinting and/or for holding open of a hollow organ with a tubular and self-expanding netting made from filaments (11; 11'; 26; 36; 45) which, in a collapsed state, is stretched in an axial direction (13) and in an expanded state is shortened in the axial direction (28; 38), wherein the filaments (11; 11'; 26; 36; 45) are configured with rectangular-shaped cross-sections and are completely jacketed in rubber-elastic plastic (46) at crossing-points (12; 27; 37; 47) of the stent (10; 25; 35) in order to form a fixed joint at the crossing-point, (12; 27; 37; 47), whereby free spaces (16; 29; 39) are formed between the filaments (11; 11'; 26; 36; 45).

2. Stent of claim 1, characterized in that the netting is constructed from wire filaments, in particular from metal.

3. Stent according to one of the claims 1 or 2, characterized in that the filaments are jacketed by a rubber-elastic plastic material (19).

4. Stent according to one of the claims 1 through 3, characterized in that the filaments (11; 11'; 26; 36) can be intertwined or woven into a tubular netting opposite to their intrinsic bend.

5. Stent according to one of the claims 1 through 4, characterized in that the filaments (11; 11'; 26; 36) are provided with a hydrophilic layer (20).

6. Stent according to one of the claims 1 through 5, characterized in that the stent (10; 25; 35) is arranged at an insertion instrument in a detachable fashion.

## Revendications

1. Dilatateur en vue de la dilatation et/ou du maintien à l'état dilaté d'un organe creux, équipé d'un réseau tubulaire auto-expansible se composant de filaments (11;11';26;36;45), qui est allongé axialement (13) dans l'état rétracté et raccourci axialement (28;38) dans l'état dilaté, les filaments (11;11';26;36;45) étant de forme rectangulaire en coupe et étant gainés d'une matière plastique (46) élastique de façon solidaire aux points de croisement (12;27;37;47) du dilatateur (10; 25; 35), afin de former une articulation fixe aux points de croisement (12; 27; 37; 47), des espaces libres (16; 29; 39) étant formés entre les filaments (11; 11'; 26; 36; 45).

2. Dilatateur selon la revendication 1, caractérisé en ce que le réseau est constitué de filaments, en particulier métalliques.

3. Dilatateur selon l'une des revendications 1 ou 2, caractérisé en ce que les filaments sont gainés d'une matière plastique élastique (19).

4. Dilatateur selon l'une des revendications 1 à 3, caractérisé en ce que les filaments (11; 11'; 26; 36) peuvent être tressés ou tissés en réseau tubulaire à l'encontre de la flexion principale.

5. Dilatateur selon l'une des revendications 1 à 4, caractérisé en ce que les filaments (11; 11'; 26; 36) sont munis d'un revêtement hydrophile (20).

6. Dilatateur selon l'une des revendications 1 à 5, caractérisé en ce que le dilatateur (10; 25; 35) est disposé de façon amovible sur une sonde.
